# EUROPEAN PATENT APPLICATION

(11) **EP 0 931 633 A2**
(43) Date of publication of application: **28.07.1999**
(21) Application number: 99300452.2
(22) Date of filing: 21.01.1999
(51) Int. Cl.: B29C 41/14, B29D 31/00, B29C 41/22, A61B 19/04, B29K 75/00, B29K 9/06

(54) **Multi-layered thin-walled powder-free articles**

(30) Priority: 21.01.1998 CA 2227645
(71) Applicant: Eci Medical Technologies Inc., Bridgewater, Nova Scotia B4V 3W7 (CA)
(72) Inventor: Sharma, Pam, Montreal, Quebec H3T 1H6 (CA); Gaetz, Lisa, Nova Scotia BOT 1KO (CA); Leidner, Jacob, Toronto, Ontario M3H 1C8 (CA); Hacker, David M., Toronto, Ontario M5N 1L9 (CA)
(74) Representative: Harrison, David Christopher

(57) **Abstract**

A method for the forming of a thin-walled powder-free article in the form of a glove, and the resultant glove. The method comprises the steps of coating a glove mould with a solution of a composition of synthetic rubber e.g. styrene block copolymer, or a mixture of such polymers, in organic solvent, and drying the mould so coated to form a coating of said composition on the glove mould, and coating the coated glove mould in an aqueous emulsion of an elastomeric latex, and drying the coated glove mould so obtained.

## Description

### Field of the Invention

The present invention relates to multi-layered thin-walled articles manufactured from synthetic rubber, including chloroprene or styrene block copolymers, and especially to thin-walled articles in the form of medical gloves. Such gloves are both latex-free and powder-free gloves, and have not been subjected to chlorination or other such treatment procedures. More specifically, the present invention relates to powder-free medical gloves manufactured from chloroprene or styrene block copolymers in which the hand-contacting surface is a layer of elastomeric latex, especially polyurethane. The present invention also relates to a multiple step process for producing a glove with a polyurethane coating, such a glove being a powder-free glove.

### Background to the Invention

Thin-walled, extensible articles such as gloves have been made from natural rubber compositions for a long time. In order to obtain articles having a sufficiently high strength and elasticity from natural rubber compositions, it is necessary to vulcanize or in other ways chemically cross-link the molecular chains of the rubber, which requires the presence of curing agents, activators and/or accelerators. The presence of such reagents often gives rise to allergic reactions among the users of the articles. In addiction, all latices of natural rubber contain proteins, which in extreme cases may cause allergic reactions e.g. swelling of a person's neck and air passageways. Such allergic reactions can result in fatal cases of anaphylactic shock. Surgeons, health care workers and other persons wearing the gloves are particularly susceptible, but patients are also vulnerable during surgical or other procedures, and the families of all such persons can be subjected to airborne particles from clothing, hair or skin. In less extreme cases, other reactions occur, including hand dermatitis and hives. Consequently, substantial effort has been made to develop articles using synthetic polymers with rubber-like characteristics and which do not have the drawbacks of natural rubber.

In typical processes, articles made from synthetic polymers are manufactured from styrene block copolymers in a multiple-dip process that involves dipping a mould or former into solutions of the styrene block copolymers and drying the dip-coated mould between each dipping step. Such a process includes the use of powder. The powder is applied to the article following the final dipping step, prior to the curing of the article and the stripping of the article from the mould or former. The powder acts to reduce surface tackiness and thus facilitates the donning of the glove.

Powder has been implicated in post operative complications. Talc was an early powder of choice but was replaced with cornstarch, without eliminating problems associated with use of powders. Even though the powder is free of latex, because the articles are not manufactured from rubber i.e. latex-containing materials, health problems still develop. It was originally predicted on the basis of animal testing that the powder would be harmlessly absorbed by the body of the user or patient. However, evidence accumulated that cornstarch per se could cause post-operative problems, including adhesions, granulomas and starch granulomatous peritonitis.

As a result of the problems with gloves formed from latex-containing materials and also with gloves having powder, some hospitals have instituted powder-free policies i.e. they are requiring use of powder-free gloves. Thus, there is a need to develop processes that eliminate use of both latex and powder in the manufacture of medical gloves.

U.S. Patents 3,933,723 and 5,112,900 both relate to the use of solutions containing thermoplastic styrene-tri-block copolymers and plasticizers for the production of thin-walled rubber articles. Such solutions do not contain the proteins present in natural rubber i.e. the solutions are free of latex, and thus thin-walled rubber articles that are hypo-allergenic rubber articles may be obtained.

U.S. Patent 5,444,121 relates to thin-walled rubber articles formed from a variety of compositions of styrene-isoprene-styrene (S-I-S) tri-block copolymers and styrene-butadiene-styrene (S-B-S) tri-block copolymers and, optionally, styrene-olefin-styrene (S-O-S) tri-block copolymers.

U.S. Patent 5,112,900 and 5,407,715 describe the use of mixtures of styrene-ethylene/butylene-styrene (S-EB-S) tri-block copolymers in the manufacture of gloves.

Blends of styrene-butadiene block copolymers and styrene-isoprene block copolymers, including S-I-S and S-B-S tri-block copolymers are disclosed for medical applications, including as gloves, in published Canadian patent application 2,112,646. Gloves made from a variety of polymers, including S-I-S and S-B-S tri-block copolymers, without the use of a solvent, are disclosed in WO 94/20574.

Continuous processes for the manufacture of thin walled articles that are free of both latex and powder are still required, and would be of significant value to both the health care industry and patients.

### Summary of the Invention

Thin-walled articles, especially in the form of gloves, made from synthetic rubbers, including chloroprene and styrene block copolymers, and having an improved internal slip coating have now been found.

Aspects of this invention relate to a method that involves multiple coating of the glove mould with a solution of a composition of styrenic block copolymer in organic solvent and drying the coating between each coating, then coating the coated glove mould in an aqueous dispersion of polyurethane coating and drying the coated glove mould obtained. The resulting glove is then stripped from the mould. This process allows for continuous production of gloves and thus tends to be more economical than conventional processes employed in the manufacture of powder-free gloves. The coating process may be a dip coating or other coating process.

In particular, an aspect of the present invention provides a method for the forming of a thin-walled powder-free article in the form of a glove, comprising the steps of:
(a) coating a glove mould with a solution of a synthetic rubber composition, especially chloroprene or styrene block copolymer, or mixture of such polymers, in organic solvent, and drying the mould so coated to form a coating of said composition on the glove mould,
(b) coating said coated glove mould in an aqueous emulsion of an elastomeric latex, and drying the coated glove mould so obtained.

In a preferred embodiment of the present invention, step (a) is repeated at least once, and especially 2-4 times, with the coating being dried after each coating of the mould, prior to step (b), so as to obtain a thicker coating on the mould.

In a further embodiment, the elastomeric latex is a polyurethane.

In a still further embodiment, the elastomeric latex contains a crosslinking agent, especially a polyfunctional aziridine.

In another embodiment, the article is stripped from the mould by everting the article.

Another aspect of the present invention provides a thin-walled article comprising:
(a) a layer of a composition of a synthetic rubber, especially chloroprene or styrene block copolymer, or mixtures thereof, and
(b) a layer of a composition of an elastomeric latex, said layer of (b) being the internal layer of the glove.

In a preferred embodiment of the method and thin-walled article of the present invention, the synthetic rubber is styrene block copolymer, preferably styrene tri-block copolymer, especially styrene tri-block copolymer selected from styrene-isoprene-styrene tri-block copolymers, styrene-butadiene-styrene tri-block copolymers, styrene-ethylene/butylene-styrene tri-block copolymers and, optionally styrene-olefin-styrene tri-block copolymers, and mixtures thereof.

In a further embodiment, the layer of elastomer latex is a polyurethane.

In a still further embodiment, the layer of elastomeric latex is cross-linked.

In another embodiment, the thin-walled article is a glove.

In a preferred embodiment of the glove of the present invention, the powder-free glove may be easily stripped from the mould, is not tacky and exhibits good donning characteristics.

### Detailed Description of the Invention

The present invention relates to a method in which a mould (also known as a former), typically a mould in the form of a glove, is coated with synthetic rubber, especially chloroprene or styrene block copolymer. The invention will be particularly described with reference to the use of styrene block copolymers.

In the method, the styrene block copolymer is dissolved in organic solvent. The styrene block copolymers for use in such a process are known, and include styrene-isoprene-styrene(S-I-S) tri-block copolymers, styrene-butadiene-styrene (S-B-S) tri-block copolymers, styrene-ethylene/butylene-styrene (S-EB-S) tri-block copolymers, and optionally including styrene-olefin-styrene (S-O-S) tri-block copolymers. It may be particularly advantageous to use mixtures of such polymers. Related styrene-olefin-di-block copolymers, e.g. styrene-isoprene and styrene-butadiene di-block copolymers may also be used. The present invention will be generally described with reference to the use of styrene tri-block copolymers.

If mixtures of S-I-S and S-B-S tri-block copolymers are used, the amount of S-I-S tri-block copolymer may be varied from about 10% by weight to about 100% by weight. S-O-S tri-block copolymers may be added to the composition. S-EB-S tri-block copolymers may be used as the sole styrene block copolymer, or in admixture with other styrene block copolymers e.g. as a replacement for S-B-S tri-block copolymer in the composition. The use of such tri-block copolymers and mixtures thereof will be understood by persons skilled in the art.

As organic solvent for the styrene block copolymer, any inert solvent for the copolymer may be used. A solvent primarily consisting of aliphatic hydrocarbons is preferred, as vapours from aliphatic hydrocarbons tend to have lower toxicity than, for instance, halogen-containing or aromatic solvents. To obtain sufficiently high dissolution of the styrene block copolymer and good process characteristics, the solvent may advantageously contain a small amount of aromatic compounds, for instance up to 10% by weight of a solvent mainly consisting of aliphatic hydrocarbons may be aromatic hydrocarbons. It is furthermore preferred that the aliphatic hydrocarbons have a boiling point between 95°C and 140°C. Depending on the particular blend of styrene block copolymers, the organic solvent is preferably used in amounts of 400-1200% by weight, based on the weight of styrene block copolymer.

It is to be understood that the solution for the preparation of a thin walled article according to the invention may have only one type of styrene block copolymer or a blend of different types of styrene block copolymers, the composition of styrene block copolymers affecting the properties of the thin-walled articles that are obtained. Moreover, each type of styrene block copolymer may contain more than one variant of that type of styrene block copolymer to attain a desired viscosity of the solution and/or to attain the desired mechanical properties of the thin-walled article. In particular, and as an example, if the styrene block copolymer was S-I-S triblock copolymer, the solution may contain several variants of the S-I-S styrene tri-block copolymer with different styrene contents and/or with different solution viscosities.

A particular tri-block copolymer e.g. S-I-S, may be characterized by the viscosity of a predetermined concentration in a solvent and the content of the end blocks in the tri-block copolymer. Thus, a S-I-S tri-block copolymer may be characterized by its solution viscosity in a 25% by weight solution in toluene at a specified temperature and by its styrene content. For instance, a preferred S-I-S tri-block copolymer has a styrene content of from 10-30% by weight and a solution viscosity of a 25% by weight solution of the copolymer in toluene at 25°C of 0.5-5 Pa.s.

Examples of S-I-S tri-block copolymers are those sold under the name Kraton^{TM} TR1107 or Kraton TR1111, which have styrene contents of 15% and 22% respectively, and solution viscosities in a 25% by weight solution in toluene at 25°C of 1.6 and 1.4 Pa.s, respectively. Alternatively, the S-I-S tri-block copolymers may be of the type sold under the name Dexco^{TM} 4111, which has a styrene content of about 18%, and a solution viscosity of a 25% by weight solution of the copolymer in toluene at 25°C of 0.7-1.0 Pa.s.

Similarly, S-B-S tri-block copolymers with a range of solution viscosities may be used, for instance with a solution viscosity of a 25% by weight solution of copolymer in toluene at 25°C of 0.5-30 Pa.s. The styrene content is preferably between 20 and 40% by weight. Examples of S-B-S tri-block copolymers include Kraton TR1101 with a styrene content of 31% by weight and a solution viscosity of a 25% by weight solution of copolymer in toluene at 25°C of 4.0 Pa.s; Kraton TR1102 with a styrene content of 29% by weight and a solution viscosity of a 25% by weight solution of copolymer in toluene of 1.2 Pa.s., and Kraton 1184 with a styrene content of 30% by weight and a solution viscosity of a 25% by weight solution of copolymer in toluene at 25°C of 20.0 Pa.s. Other examples of S-B-S tri-block copolymers are Dexco 2518 and Dexco 8508D. S-B-S tri-block copolymers typically have a very high modulus which results in an article with higher strength but a lower degree of elasticity.

Styrene-ethylene/butylene-styrene (S-EB-S) tri-block copolymer may also be used, preferably with a styrene content of 10-30% by weight and a solution viscosity of a 25% by weight solution of copolymer in toluene at 25°C of 1.0-10 Pa.s.

In an embodiment, the composition comprises 10-25% by weight S-B-S tri-block copolymer, 10-25% by weight S-EB-S tri-block copolymer and preferably greater than 75% by weight S-I-S tri-block copolymer. As an example of a mixture of such copolymers, the rubber thin-walled article should be comprised of about one part S-B-S tri-block copolymer, about one part S-EB-S tri-block copolymer and about 6 parts S-I-S tri-block copolymer. The thin-walled article comprising this copolymer blend preferably contains up to about 30% by weight of a naphthenic plasticizer, based on the copolymer blend.

A rubber article in accordance with the present invention with a greater ozone resistance may be provided by replacing a part of the S-I-S tri-block copolymer or part of the S-B-S tri-block copolymer by styrene-olefin-styrene (S-O-S) tri-block copolymers. A polymerized olefin is saturated, i.e., contains no double bonds, and is therefore less sensitive towards ozone. Such a substitution may require that the solvent, apart from the aliphatic hydrocarbons, contain a lower level of aromatic hydrocarbons in order to attain the desired solution and in order to secure the development of a polymer matrix in which the various polymer chains form an interpenetrating molecular network.

As noted above, the styrene block copolymers are a preferred synthetic rubber of the method of the invention. Other synthetic rubbers may be used e.g. chloroprene.

In the present invention, the solution for the production of thin-walled rubber articles typically contains commonly used additives, such as antioxidants, slip agents, and anti-blocking agents. Zinc dithiocarbamate and 4, 4'-butylidene bis (6-tert-butyl-m-cresol) are examples of suitable antioxidants, present in quantities not greater than about 0.5 weight percent. Slip agents, which are used to assist in mould release, may be of the modified fatty acid ester type and may be present in quantities typically not greater than 3.0 weight percent. Anti-blocking agents, which help prevent tackiness, are typically waxes, and may be present in quantities not greater than 5.0 weight percent.

In the method of the invention, the mould is coated with the organic solution of the synthetic rubber e.g. chloroprene or styrene block copolymer. The method will be particularly described with respect to a dip-coating process, but it is understood that other coating processes may be used.

Thus, in an embodiment of the invention, the mould is coated with an organic solution of styrene tri-block copolymers by dipping the mould into the solution. The dip-coated mould is then removed from the solution and dried, usually dried in air at about the solution temperature. In a typical process, the mould is dip-coated more than once, and usually using a process that is operated continuously, i.e. the mould is dip-coated in a first solution, air dried, dip-coated in a second solution and air dried, and so forth, with the mould being suspended from a continuous belt so that it passes through the coating baths and through the air drying steps in sequence under controlled conditions. It is understood that the mould may be dip-coated a number of times e.g. up to at least four times, or more. The solutions in each coating bath used may be identical, or different solutions may be used e.g. solutions of different composition.

Subsequent to the steps of coating the mould with the styrene block copolymers or other synthetic rubber as described herein, the coated mould is subjected to a step in which it is coated with an aqueous emulsion of an elastomeric latex, especially polyurethane. It is to be understood that the coating of styrene block copolymers or other synthetic rubber on the coated mould would need to be sufficiently dry to be able to effect the coating with the aqueous emulsion. In particular, the coating must be sufficiently dry so as to avoid solvent entrapment when the polyurethane layer is applied. Examples of drying steps including drying in a heating tunnel at temperatures in a range of 50°C to 120°C, depending on the line speed, for a maximum of 20 minutes, and/or use of increased airflow around the formers to increase the volatility of the hydrocarbon solvent and thus facilitate more evaporation of the hydrocarbon solvent from the polymer substrate.

The coating may be air dried at ambient temperatures prior to drying in the oven. Ineffective drying of the polymer substrate may result in bubbling or solvent popping in the thicker areas of the glove. The aqueous emulsion is formed from an elastomeric latex, and typically also contains a reducing agent, a polyfunctional liquid crosslinker and slip enhancing additives. The latter include slip additives for enabling the donning of the glove when hands are dry i.e. so-called dry-donning additives, and slip additives for enabling the donning of the glove when hands are wet i.e. so-called wet-donning additives.

The elastomeric latex is a synthetic elastomeric latex, and does not contain rubber compounds and especially does not contain rubber latex. A variety of synthetic elastomeric latices may be used, with polyurethane being preferred. The elastomeric latex must exhibit good adhesion to the dried composition of styrene block copolymers previously dip-coated from the solvent-based solutions described above in the fabrication of the glove. In addition, the elastomeric latex must exhibit high elongation to permit stretching of the article e.g. glove, during donning of the glove. Furthermore, the elastomeric latex must be acceptable for contact with skin, including complying with the requirements of any pertinent governmental or other regulations.

Examples of elastomeric latices include aliphatic polyurethanes e.g. NeoRez^{TM} R962, NeoRez R967 and Solcoute 10511-3-35 aqueous polyurethane dispersions. NeoRez R962 dispersion is a non-tonic dispersion and the latter two dispersions are anionic dispersions. Such aliphatic polyurethanes are prepared and are available as an aqueous dispersion containing 30-40% solids.

Reducing agents, if present, are used to dilute the solution containing the aqueous dispersion and to provide appropriate drying and wetting properties. Alcohol e.g. methanol or ethanol, may be added to the dispersion in amounts that do not affect the stability of the dispersion e.g. less than 60% of the total solvent blend in the dispersion. Water may also be added to the diapersion in amounts that do not influence the wetting of the polymer substrate by the coating.

A crosslinking agent such as a polyfunctional aziridine crosslinker may also be added to improve the alcohol resistance of the polyurethane coating. Addition of the crosslinker increases the crosslinking of the polyurethane film itself, thereby making it more chemically resistant.

Fluorosurfactants e.g. 3M FC430 or Du Pont Zonyl^{TM} FSN, may be added into the aqueous dispersion to improve wetting properties of the aqueous dispersion on the styrenic block copolymer substrate.

The aqueous dispersion contains both dry slip additives and wet slip additives. Examples of the dry slip additives include paraffinic waxes, hydrocarbon polyolefins and/or fluorinated polyolefins. Examples of such additives include Microspersion^{TM} 114, Microspersion 411 and Microspersion 523, all available from Micro Powders, Inc., Michem Lube^{TM} 160 and Michem Lube 180 which are available from Michelman Inc. Microspersion 114 is understood to be a synthetic wax. Microspersions 411 and 523 are understood to be dispersions of polyethylene and polytetrafluoroethylene (PTFE). Michem Lube 160 is understood to be a dispersion of carnauba wax, and Michem Lube 180 to be a dispersion of a mixture of carnauba and paraffin waxes.

Examples of damp (wet) slip additives are polyethylene glycols, polyethylene oxides and sulfoacetates. For polyethylene glycols, examples include PEG 200, PEG 400, PEG 3400 and PEG 8000. For polyethylene oxides, examples include Polyox^{TM} N10, Polyox N80, Polyox N750 and Polyox N3000, all of which are available from Union Carbide. An example of a sulfoacetate is Lanthanol LAL, available from Stepan, which is sodium lauryl sulfoacetate and is an anionic surfactant.

In an embodiment of the aqueous dispersion used in the invention, each additive was incorporated with agitation into a blend of 25% aliphatic polyurethane and 75% solvent. Additions of slip enhancing additives are based on total resin solids (TRS). To the dispersion may be added 5-20% by weight of dry additive, 1-5% by weight of liquid crosslinker, and 0.1-15% by weight of damp additive, especially less than 5% by weight thereof. The viscosity of the coating itself should be within the range of 0.01-0.02 Pa.s and the percentage by weight of solids in the coating is preferably within the range 9-10% to facilitate coverage of the polymer substrate.

A crosslinking agent, e.g. a polyfunctional aziridine crosslinker, may be added to the coating in an amount effective to improve the alcohol resistance of the coating layer. Addition of the crosslinking agent may be important, in that alcohol-based disinfectants are commonly used in some countries as a donning preparation for the donning of gloves. In the absence of the crosslinking agent, the coating may exhibit poor resistance to alcohol-based disinfectants. As exemplified below, use of a crosslinker results in substantial improvement in alcohol resistance. It should be noted that in water-borne systems e.g. the polyurethane coating formulation, polyfunctional asiridine crosslinkers tend to slowly hydrolyse and become inactive, which must be taken into account when replenishing the coating solution or maintaining it at desired concentration levels.

The dwell time for the coating dip is dependent on the uniformity of thickness of the coating, and is dependent on line speed. However, typical times may be 15-30 seconds.

If a dip-coating process is used, the former (mould) should be slowly removed from the coating and then rotated to assist in uniform distribution of the coating over the polymer substrate. The gloves are air dried at ambient temperature prior to further drying in a forced air oven. This facilitates curing of the polymer composition. The gloves are then allowed to cool at room temperature and stripped from the form at which time the gloves are everted.

The method of the invention provides a powder-free glove that strips (everts) easily from the mould, that is not tacky, that possesses good dry and damp donning characteristics, and that can be produced in a continuous fashion without interruption. Moreover, the powder-free gloves do not require treatment by chlorination or other methods in order to attain acceptable characteristics, including wet and dry donning properties.

The present invention is illustrated by the following examples.

### Example I

Samples were prepared with a polyurethane dispersion containing some of the above examples of additives. Both dry and damp skin lubricity were subjectively evaluated based on a scale of 1-5, 1 indicating the inner surface of the glove is tacky, and 5 indicating that the internal slip is excellent.

The results obtained were as follows:

**TABLE I**

| | Coating Formulation | Dry Skin Lubricity | Damp Skin Lubricity |
|---|---|---|---|
| (1) | NeoRez R962 | | |
| | Microspersion 114 (20%) | 5 | 2 |
| | Polyox N3000 (5%) | | |
| (2) | NeoRez R962 | | |
| | Microspersion 114 (20%) | 4 | 2 |
| | Polyox N10 (5%) | | |
| (3) | NeoRez R962 | | |
| | Microspersion 114 (20%) | 5 | 3 |
| | PEG 200 (5%) | | |
| (4) | NeoRez R962 | | |
| | Microspersion 114 (20%) | 5 | 5 |
| | Lanthanol LAL (5%) | | |

In further tests, for formulations (1)-(3), the concentration of the damp additive was increased to 15% with no improvement in damp slip. In separate tests, the concentration of the Lanthanol LAL sulfoacetate in formulation (4) was decreased to 1% with similar results.

### Example II

A polyurethane coating system as described herein was coated onto a former. In separate experiments, the coating system was formulated (i) with 2% by weight of CX-100 Crosslinker polyfunctional aziridine crosslinker from Zeneca Resins, and (ii) without crosslinking agent.

To test the alcohol resistance of the coating, a Q-TIP^{®} was soaked in an alcohol-based disinfectant and rubbed back and forth across the coated surface. The number of double rubs to dissolve the coating was recorded.

The results obtained were as follows:

**TABLE II**

| Alcohol-based Disinfectant* | # of Double Rubs to Dissolve Coating | |
|---|---|---|
| | Control ** | Add Crosslinker (2%) |
| Sterillium | <10 | >50 |
| Spitacid | <10 | >50 |
| Desderman | <10 | >50 |

| | | |
|---|---|---|
| * Commercially available | | |
| ** Without added CX-100 Crosslinker | | |

The results show that resistance to alcohol increased by at least 400%.

## Claims

**1.** A method for the forming of a thin-walled powder-free article in the form of a glove, comprising the steps of:
(a) coating a glove mould with a solution of a synthetic rubber composition in organic solvent, and drying the mould so coated to form a coating of said composition on the glove mould,
(b) coating said coated glove mould in an aqueous emulsion of an elastomeric latex, and drying the coated glove mould so obtained.

**2.** The method of Claim 1 in which the elastomeric latex is polyurethane.

**3.** The method of Claim 1 or Claim 2 in which the elastomeric latex is cross-linked.

**4.** The method of Claim 3 in which the polyurethane is cross-linked with polyfunctional aziridine.

**5.** The method of any one of Claims 1-4 in which the synthetic rubber is polychloroprene.

**6.** The method of any one of Claims 1-4 in which the synthetic rubber is a styrene block copolymer.

**7.** The method of Claim 1 in which step (a) is repeated at least once so as to obtain a thicker coating of said synthetic rubber composition on the mould.

**8.** The method of Claim 6 in which the styrene block copolymer is styrene tri-block copolymer.

**9.** The method of Claim 8 in which styrene tri-block copolymer is selected from styrene-isoprene-styrene tri-block copolymers, styrene-butadiene-styrene tri-block copolymers, styrene-ethylene/butylene-styrene tri-block copolymers and, optionally styrene-olefin-styrene tri-block copolymers, and mixtures thereof.

**10.** The method of any one of Claims 1-9 in which the article is stripped from the mould by everting the article.

**11.** The method of any one of Claims 1-10 in which the resultant powder-free glove may be easily stripped from the mould, is not tacky and exhibits good donning characteristics.

**12.** The method of any one of Claims 1-11 in which the organic solvent of step (a) is selected from aliphatic hydrocarbons and blends of aliphatic and aromatic hydrocarbons, such organic solvent having a boiling point between 95°C and 140°C, the amount of organic solvent being 400-1200% by weight of the sytrene block copolymer.

**13.** The method of any one of Claims 1-12 in which the aqueous emulsion contains dry slip and wet slip additives for donning of the gloves.

**14.** A thin-walled powder-free article comprising:
(a) a layer of a composition of a synthetic rubber, and
(b) a layer of an elastomeric latex, said layer of (b) being the internal layer of the glove.

**15.** A thin-walled powder-free article comprising:
(a) a layer of a composition of a styrene block copolymer, or mixtures thereof, and
(b) a layer of an elastomeric latex, said layer of (b) being the internal layer of the glove.

**15.** The article of claim 15 in which the article is removed from the mould by everting the article.

**17.** The article of claim 15 or claim 16 in which the styrene block copolymer is styrene tri-block copolymer.

**18.** The article of claim 17 in which the styrene tri-block copolymer is selected from styrene-isoprene-styrene tri-block copolymers, styrene-butadiene-styrene tri-block copolymers, styrene-ethylene/butylene-styrene tri-block copolymers and, optionally styrene-olefin-styrene tri-block copolymers, and mixtures thereof.

**19.** The article of any one of claims 14 to 18 in which the thin-walled article is a glove.

**20.** The article of claim 19 in which the glove may be donned by a user without use of a powder.

**21.** The article of any one of claims 14 to 20 in which the elastomeric latex is polyurethane.

**22.** The article of any one of claims 14 to 21 in which the elastomeric latex is cross-linked.

**23.** The article of claim 22 in which the polyurethane is cross-linked with polyfunctional aziridine.
